Europäisches Patentamt

European Patent Office

Office européen des brevets

(11) Veröffentlichungsnummer: **0 467 125 B1**

(12) **EUROPÄISCHE PATENTSCHRIFT**

(45) Veröffentlichungstag der Patentschrift: **07.09.94**

(51) Int. Cl.5: **C07C 263/10, C07C 263/20**

(21) Anmeldenummer: **91110635.9**

(22) Anmeldetag: **27.06.91**

(54) **Verfahren zur Herstellung von Mischungen aus Diphenylmethan-diisocyanaten und Polyphenylpolymethylen-polyisocyanaten mit einer verminderten Iodfarbe.**

(30) Priorität: **07.07.90 DE 4021712**

(43) Veröffentlichungstag der Anmeldung:
**22.01.92 Patentblatt 92/04**

(45) Bekanntmachung des Hinweises auf die
Patenterteilung:
**07.09.94 Patentblatt 94/36**

(84) Benannte Vertragsstaaten:
**AT BE CH DE DK ES FR GB IT LI NL SE**

(56) Entgegenhaltungen:
EP-A- 0 114 970      EP-A- 0 183 976
EP-A- 0 445 602      DE-A- 2 249 375
DE-A- 3 306 845      US-A- 2 999 873

(73) Patentinhaber: **BASF Aktiengesellschaft
Carl-Bosch-Strasse 38
D-67063 Ludwigshafen (DE)**

(72) Erfinder: **Scherzer, Dietrich, Dr.
Prager Strasse 33
W-6700 Ludwigshafen (DE)**
Erfinder: **Minges, Roland, Dr.
Auf der Setz 23
W-6718 Gruenstadt (DE)**
Erfinder: **Bruchmann, Bernd, Dr.
Giselherstrasse 79
W-6700 Ludwigshafen (DE)**
Erfinder: **Heider, Wolfgang
Albert-Einstein-Allee 16
W-6703 Limburgerhof (DE)**
Erfinder: **Van Pee, Willy, Dr.
Bunderbeeklaan 18
B-2080 Kapellen (BE)**
Erfinder: **Keller, Peter, Dr.
Winzerweg 2
W-6945 Hirschberg (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

**Beschreibung**

Erfindungsgegenstand ist ein Verfahren zur Herstellung von Mischungen aus Diphenylmethan-diisocyanaten und Polyphenyl-polymethylen-polyisocyanaten, sogenanntes Roh-MDI, mit einer reduzierten Iodfarbzahl durch Umsetzung der entsprechenden Mischungen aus Diphenylmethan-diaminen und Polyphenyl-polymethylen-polyaminen, sogenanntes Roh-MDA, mit Phosgen in Gegenwart mindestens eines inerten organischen Lösungsmittels, wobei man der Reaktionsmischung nach beendeter Phosgenierung ein- oder mehrwertige Polyoxyalkylenalkohole oder Mischungen davon in einer wirksamen Menge einverleibt.

Roh-MDI, einer der technisch bedeutendsten Ausgangsstoffe zur Herstellung von Polyisocyanat-polyadditionsprodukten, beispielsweise Urethan- oder Urethan- und Isocyanuratgruppen enthaltenden Schaumstoffen, und von 4,4'-Diphenylmethan-diisocyanat, einer wichtigen Aufbaukomponente zur Herstellung von Polyurethan(PU)-Elastomeren, -Fasern, -Dichtungsmassen, -Klebstoffen u.a. wird bekanntermaßen hergestellt durch Phosgenierung von Roh-MDA, üblicherweise in Gegenwart eines inerten organischen Lösungsmittels. Roh-MDA wird seinerseits erhalten durch Kondensation von Anilin und Formaldehyd in Gegenwart von sauren Katalysatoren, wobei in Abhängigkeit von den gewählten Mengenverhältnissen der Ausgangsstoffe und den Reaktionsbedingungen sowie den unterschiedlichen Verfahren der prozentuale Anteil an Diphenylmethan-diaminen und den homologen Polyphenyl-polymethylen-polyaminen sowie ihren Isomeren gesteuert werden kann (Kunststoff-Handbuch, Band 7, Polyurethane, 1. Auflage 1966 und 2. Auflage 1983, Carl-Hanser-Verlag, München, Wien). Wird die Kondensation von Anilin und Formaldehyd z.B. in Gegenwart von schwach sauren Katalysatoren durchgeführt, so erhält man Roh-MDA-Gemische mit einem relativ hohen Anteil an 2,2'- und 2,4'-Diamino-diphenylmethanen, während Roh-MDA-Gemische mit einem großen Gehalt an 4,4'-Diamino-diphenylmethan und gleichzeitig geringem Anteil an 2,4'-Diamino-diphenylmethan nur in Gegenwart von größeren Mengen stark saurer Katalysatoren, vorzugsweise von starken Mineralsäuren, wie z.B. Salzsäure, hergestellt werden können.

Das Verhältnis von Diamino-diphenylmethan-Isomeren zu den höheren Homologen im Roh-MDA ist ferner abhängig vom Anilin-Formaldehyd-Verhältnis und der Kondensationstemperatur, wobei größere Anilin-Formaldehyd-Verhältnisse und niedrige Kondensationstemperaturen hohe Diamino-diphenylmethangehalte ergeben (CA-A-700 026).

Nachteilig an diesen Herstellungsverfahren, die in einer Vielzahl von Literatur- und Patentpublikationen beschrieben werden, ist die Bildung von mehr oder weniger stark gefärbten Roh-MDA, deren Farbe von schwarz über dunklere und hellere Brauntöne bis zu ocker variieren kann. Nachteilig ist ferner, daß diese Verfärbungen auch durch die anschließende Phosgenierung zur Herstellung der entsprechenden Roh-MDI nicht oder nur unzureichend vermindert werden und das gebildete Roh-MDI nicht durch Destillation gereinigt werden kann. Diese unerwünschte Verfärbung wird außerdem in den Folgeprodukten wirksam, so daß auch die aus gefärbtem Roh-MDI hergestellten gegebenenfalls zellhaltigen Polyisocyanat-Polyadditionsprodukte nicht farblos sind. Obgleich die Eigenfarbe der Polyisocyanat-Polyadditionsprodukte deren mechanische Eigenschaften nicht negativ beeinflußt, werden vom Verbraucher im wesentlichen farblose Produkte gewünscht.

Es hat daher nicht an Versuchen gefehlt, die Verfärbungen von Roh-MDI zu vermindern und die hergestellten Polyisocyanate durch geeignete Verfahrensmaßnahmen oder Zusatzstoffe zu stabilisieren.

Nach Angaben der US-A-2 885 420 können organische Polyisocyanate gegen eine Verfärbung durch die Zugabe von 0,01 bis 0,5 Gew.-%, bezogen auf das Polyisocyanatgewicht, eines aromatischen, cycloaliphatischen oder aliphatischen Ethers oder Thioethers stabilisiert werden.

Zur Beseitigung von als Gelbildungskatalysatoren wirkenden Verunreinigungen in organischen Diisocyanatlösungen, werden diesen gemäß DE-A-1 280 855 (GB 1 097 219) etwa 0,001 bis 1 Gew.-%, bezogen auf das Gewicht des Diisocyanats, Phosphorsäure zugesetzt.

Die GB-B-1 465 014 beschreibt den Zusatz von Glycidol in einer Menge von 0,001 bis 0,25 Gew.-%, bezogen auf das Diisocyanatgewicht, zur Verbesserung der Lagerstabilität von destillierten Diphenylmethandiisocyanaten.

Die EP-B-0 183 976 (US-A-4 677 221) betrifft ein Verfahren zur Herstellung von wärmefarbbeständigen (cyclo)aliphatischen Diisocyanaten, wobei man technisches Diisocyanat mit aliphatisch und/oder cycloaliphatisch gebundenen Isocyanatgruppen in Gegenwart von 0,1 bis 3 Gew.-% einer in dem Diisocyanat löslichen Verbindung, welche mindestens 3 Gew.-% an Struktureinheiten der Formel -NH-CO- aufweist, während eines Zeitraums von bis zu 5 Stunden auf eine Temperatur von 100 bis 220°C erhitzt und anschließend das so behandelte Diisocyanat durch Destillation reinigt. Das Verfahren ist nicht auf die Behandlung von Roh-MDI übertragbar, da, wie bereits ausgeführt wurde, dieses nicht destillierbar ist.

Nach Angabe der US-A-4 465 639 werden Roh-MDI nach beendeter Phosgenierung, aber vor der vollständigen Abtrennung des Phosgens, 0,1 bis 5 Gew.-% Wasser, bezogen auf das Polyisocyanatgewicht der Reaktionsmischung, einverleibt. Durch diese Maßnahme kann die Farbe des Roh-MDI und der daraus hergestellten PU-Schaumstoffe aufgehellt werden. Ferner wird der Anteil an höhermolekularen MDI-Homologen im Roh-MDI beträchtlich erniedrigt und ihre Viskosität reduziert. Obgleich auf diese Weise die Jodfarbzahl des Roh-MDI gesenkt werden kann, sind mit dieser Methode auch erhebliche Nachteile verbunden. Durch die Gegenwart von Wasser wird die korrodierende Wirkung der Chlor, Chlorwasserstoff und Phosgen enthaltenden Reaktionsmischung auf die Apparate der Produktionsanlage beträchtlich verstärkt und dadurch das Leckagerisiko, verbunden mit einem Ausbruch von toxischem Phosgen oder einer phosgenhaltigen Reaktionsmischung, erhöht. Aus Sicherheitsgründen wird daher Feuchtigkeit in jeder Form bei der Phosgenierung zweckmäßigerweise im wesentlichen vollständig ausgeschlossen.

Die Aufgabe der vorliegenden Erfindung bestand darin, unter Vermeidung der genannten Nachteile die Jodfarbzahl von Roh-MDI dem durch Wasserzugabe erreichbaren Niveau anzugleichen oder noch stärker zu vermindern, wobei insbesondere auf den Zusatz von Wasser verzichtet werden sollte.

Diese Aufgabe konnte überraschenderweise gelöst werden durch den Zusatz von ein- oder/und mehrwertigen Polyoxyalkylenalkoholen zu der phosgenhaltigen Reaktionsmischung nach beendeter Phosgenierung.

Gegenstand der Erfindung ist somit ein Verfahren zur Herstellung von Roh-MDI mit einer reduzierten Jodfarbzahl durch Umsetzung der entsprechenden Roh-MDA mit Phosgen in Gegenwart mindestens eines inerten organischen Lösungsmittels bei erhöhter Temperatur,
nach beendeter Phosgenierung Abtrennung des überschüssigen Phosgens und Lösungsmittels und thermischer Behandlung des erhaltenen Reaktionsprodukts,
das dadurch gekennzeichnet ist, daß man der Reaktionsmischung nach beendeter Phosgenierung ein- oder mehrwertige Polyoxyalkylenalkohole, vorzugsweise mehrwertige Polyoxyalkylenalkohole mit einer Funktionalität von 2 bis 8 oder Mischungen aus ein- und mehrwertigen Polyoxyalkylenalkoholen in einer wirksamen Menge einverleibt.

Durch den erfindungsgemäß angewandten Zusatz der ein- und/oder mehrwertigen Polyoxyalkylenalkohole kann die Jodfarbzahl des Roh-MDI beträchtlich gesenkt werden, z.B. auf Werte von kleiner als 60, vorzugsweise von 35 bis 20 und kleiner.

Die nach dem erfindungsgemäßen Verfahren hergestellten Mischungen aus Diphenylmethan-diisocyanaten (MDI) und Polyphenyl-polymethylen-polyisocyanaten besitzen ferner vorteilhafterweise einen MDI-Isomerengehalt von 30 bis 90 Gew.-%, vorzugsweise von 30 bis 70 Gew.-%, einen NCO-Gehalt von 31±2 Gew.-%, vorzugsweise von 31±1,0 Gew.-%, jeweils bezogen auf das Roh-MDI-Gewicht, und eine Viskosität von maximal 2000 mPa•s, vorzugsweise von 40 bis 350 mPa•s, gemessen bei 23°C.

Roh-MDI mit solchen Isomeren- und Homologenzusammensetzungen können, wie bereits ausgeführt wurde, durch Phosgenierung von Roh-MDA mit entsprechenden Produktzusammensetzungen in Gegenwart mindestens eines inerten organischen Lösungsmittels nach bekannten Verfahren hergestellt werden.

Geeignete Roh-MDA werden vorteilhafterweise erhalten durch Kondensation von Anilin und Formaldehyd in einem Molverhältnis von 6 bis 1,6 : 1, vorzugsweise von 3 bis 1,9 : 1 und einem Molverhältnis von Anilin zu sauren Katalysatoren von 1 : 0,98 bis 0,01, vorzugsweise 1 : 0,8 bis 0,2.

Der Formaldehyd wird vorzugsweise in Form einer wäßrigen Lösung, z.B. als handelsübliche 30 bis 50 gew.-%ige Lösung, verwendet.

Als saure Katalysatoren haben sich Protonendonatoren, wie z.B. saure Ionenaustauscherharze oder starke organische und vorzugsweise anorganische Säuren bewährt. Als starke Säuren sind hierbei solche mit einem pKs-Wert kleiner als 1,5 - bei mehrbasischen Säuren gilt dieser Wert für die erste Wasserstoffdissoziation - zu verstehen. Beispielhaft genannt seien Salzsäure, Schwefelsäure, Phosphorsäure, Fluorsulfonsäure und Oxalsäure. Chlorwasserstoff kann auch gasförmig eingesetzt werden. Vorzugsweise zur Anwendung kommt wäßrige Salzsäure in Konzentrationen von etwa 25 bis 31 Gew.-%.

In Betracht kommende Verfahren zur Roh-MDA-Herstellung werden beispielsweise beschrieben in CA-A-700 026, DE-B-22 27 110 (US-A-4 025 557), DE-B-22 38 920 (US-A-3 996 283), DE-B-24 26 116 (GB-A-1 450 632), DE-A-12 42 623 (US-A-3 478 099), GB-A-1 064 559 und DE-A-32 25 125.

Als andere Ausgangskomponente zur Herstellung von Roh-MDI wird Phosgen verwendet. Das gasförmige Phosgen kann als solches oder in Verdünnung mit unter den Reaktionsbedingungen inerten Gasen, wie Stickstoff, Kohlenmonoxid u.a. eingesetzt werden. Das Molverhältnis von Roh-MDA zu Phosgen wird zweckmäßigerweise so bemessen, daß pro $NH_2$-Gruppe 1 bis 10 Mol, vorzugsweise 1,3 bis 4 Mol, Phosgen in der Reaktionsmischung vorliegen.

Als inerte organische Lösungsmittel kommen Verbindungen in Betracht, in welchen das Roh-MDA und das Phosgen mindestens teilweise löslich sind.

Als Lösungsmittel vorzüglich bewährt haben sich chlorierte, aromatische Kohlenwasserstoffe, beispielsweise Monochlorbenzol, Dichlorbenzole wie z.B. o-Dichlorbenzol, p-Dichlorbenzol, Trichlorbenzole, die entsprechenden Toluole und Xylole, Chlorethylbenzol, Monochlordiphenyl, $\alpha$- bzw. $\beta$-Naphthylchlorid und Phthalsäuredialkylester, wie iso-Diethylphthalat. Insbesondere Anwendung finden als inerte organische Lösungsmittel Monochlorbenzol, Dichlorbenzole oder Mischungen dieser Chlorbenzole. Die Lösungsmittel können einzeln oder als Gemische verwendet werden. Zweckmäßigerweise wird ein Lösungsmittel verwendet, das einen niedrigeren Siedepunkt besitzt als die MDI-Isomeren, damit das Lösungsmittel leicht durch Destillation vom Roh-MDI abgetrennt werden kann. Die Menge an Lösungsmittel wird zweckmäßig so bemessen, daß die Reaktionsmischung einen Isocyanatgehalt von 2 bis 40 Gew.-%, vorzugsweise zwischen 5 und 20 Gew.-%, bezogen auf das Gesamtgewicht der Reaktionsmischung, aufweist.

Das Roh-MDA kann als solches oder gelöst in organischen Lösungsmitteln zur Anwendung kommen. Insbesondere verwendet man jedoch Roh-MDA-Lösungen mit einem Amingehalt von 2 bis 40 Gew.-%, vorzugsweise von 5 bis 20 Gew.-%, bezogen auf das Gesamtgewicht der Aminlösung.

Zur Verminderung der Iodfarbzahl werden der phosgenhaltigen Reaktionsmischung erfindungsgemäß ein- oder vorzugsweise mehrwertige Polyoxyalkylenalkohole oder Mischungen davon in einer wirksamen Menge einverleibt. Geeignete Polyoxyalkylenalkohole besitzen zweckmäßigerweise eine Hydroxylzahl von 20 bis 1800, vorzugsweise von 100 bis 1100 und insbesondere von 380 bis 800 und die mehrwertigen Polyoxyalkylenalkohole weisen eine Funktionalität vorzugsweise von 2 bis 8 und insbesondere von 2 bis 3 auf. Vorzüglich bewährt haben sich Polyoxyalkylenalkohole, die in den inerten organischen Lösungsmitteln zur Herstellung des Roh-MDI's, vorzugsweise Monochlorbenzol, Dichlorbenzole oder Mischungen davon, in den erforderlichen wirksamen Mengen zumindest teilweise, vorzugsweise jedoch vollständig löslich sind.

Die Polyoxyalkylenalkohole können nach bekannten Verfahren, beispielsweise durch anionische Polymerisation mit Alkalihydroxiden, wie z.B. Natrium- oder Kaliumhydroxid oder Alkalialkoholaten, wie Natriummethylat, Natrium- oder Kaliummethylat oder Kaliumisopropylat als Katalysatoren und unter Zusatz mindestens eines Startermoleküls, das mindestens ein, vorzugsweise 2 bis 8 und insbesondere 2 oder 3 reaktive Wasserstoffatome gebunden enthält, oder durch kationische Polymerisation mit Lewis-Säuren, wie Antimonpentachlorid, Borfluorid-Etherat u.a. oder Bleicherde als Katalysatoren aus einem oder mehreren Alkylenoxiden mit 2 bis 4 Kohlenstoffatomen im Alkylenrest hergestellt werden.

Geeignete Alkylenoxide sind beispielsweise Tetrahydrofuran, 1,3-Propylenoxid, 1,2- bzw. 2,3-Butylenoxid, Styroloxid und vorzugsweise Ethylenoxid und 1,2-Propylenoxid. Die Alkylenoxide können einzeln, alternierend nacheinander oder als Mischungen verwendet werden. Als Startermoleküle kommen beispielsweise in Betracht: Alkanole mit verzweigtkettigen oder vorzugsweise linearen Alkylresten mit 1 bis 10, vorzugsweise 1 bis 4 Kohlenstoffatomen, wie z.B. Methanol, Ethanol, n- und iso-Propanol, n- und sek.-Butanol, Pentanol, Hexanol, n- und iso-Octanole, mehrwertige, vorzugsweise zwei- bis achtwertige, insbesondere zwei- und/oder dreiwertige Alkohole oder Dialkylenglykole wie z.B. Ethandiol, Propandiol-1,2 und -1,3, Diethylenglykol, Dipropylenglykol, Butandiol-1,4, Pentandiol-1,5, Hexandiol-1,6, Glycerin, Trimethylolpropan, Pentaerythrit, Sorbit und Saccharose und Wasser.

Als ein- oder mehrwertige Polyoxyalkylenalkohole kommen beispielsweise in Betracht: Polyoxytetramethylen-, Polyoxytetramethylen-polyoxypropylen-, Polyoxytetramethylen-polyoxyethylen-, Polyoxypropylen-, Polyoxypropylenpolyoxyethylen- und Polyoxyethylenalkohole. Besonders bewährt haben sich jedoch, und daher vorzugsweise Verwendung finden, Polyoxyalkylenalkohole mit einer Funktionalität von 2 bis 3 und einer Hydroxylzahl von 380 bis 800, zweckmäßigerweise solche, hergestellt aus Ethylenoxid, 1,2-Propylenoxid oder 1,2-Propylenoxid und Ethylenoxid, wobei die erhaltenen Polyoxypropylen-polyoxyethylenalkohole die Ethylenoxid- und 1,2-Propylenoxideinheiten in statistischer Verteilung oder blockweise oder in statistischer Verteilung mit Ethylenoxidendblöcken gebunden enthalten können. Insbesondere geeignet sind jedoch trifunktionelle Polyoxypropylen-polyole mit einer Hydroxylzahl von 380 bis 600, die zweckmäßigerweise unter Einsatz von Glycerin als Startermolekül hergestellt werden. Als Polyoxyalkylenalkohole im Sinne der Erfindung sind auch Dialkylenglykole zu verstehen, wobei insbesondere Diethylenglykol, Dipropylenglykol oder Mischungen davon eingesetzt werden. Die ein- oder mehrwertigen Polyoxyalkylenalkohole können einzeln oder in vorm von Mischungen verwendet werden. Geeignet sind selbstverständlich auch Mischungen aus ein- und mehrwertigen Polyoxyalkylenalkoholen.

Die zur Reduzierung der Jodfarbzahl erforderliche Menge an ein- oder mehrwertigen Polyoxyalkylenalkoholen ist abhängig von der Farbe bzw. der Menge an Verunreinigungen, die aus einer oder mehreren unbekannten Substanzen bestehen, und der Hydroxylzahl der Polyoxyalkylenalkohole und kann experimentell auf einfache Weise ermittelt werden. Gute Ergebnisse werden üblicherweise erzielt bei Verwendung von 0,1 bis 10 Gew.-%, bezogen auf das Gewicht des Roh-MDI in der Reaktionsmischung, mindestens eines Polyoxyalkylenalkohols, wobei Polyoxyalkylenalkohole mit einer Hydroxylzahl von kleiner als 380 zweckmäßigerweise in einer Menge von 2 bis 10 Gew.-%, insbesondere 2 bis 6 Gew.-%, solche mit einer Hydroxylzahl von 380 bis 800 zweckmäßigerweise in einer Menge von 0,1 bis 5 Gew.-%, insbesondere 0,2 bis 3 Gew.-% und Polyoxyalkylenalkohole mit einer Hydroxylzahl von größer als 800 zweckmäßigerweise in einer Menge von 0,1 bis 3 Gew.-%, insbesondere 0,1 bis 1,5 Gew.-%, jeweils bezogen auf das Gewicht des lösungsmittelfreien Roh-MDI, eingesetzt werden.

Die Polyoxyalkylenalkohole können in reiner oder handelsüblicher Qualität eingesetzt werden, wobei jedoch der Wassergehalt der handelsüblichen Produkte möglichst gering, zweckmäßigerweise unter 0,1 Gew.-% betragen sollte.

Nach Abtrennung des überschüssigen Phosgens und des inerten Lösungsmittels können dem ein- und/oder mehrwertige Polyoxyalkylenalkohole und/oder Reaktionsprodukte, erhältlich aus diesen ein- und/oder mehrwertigen Polyoxyalkylenalkoholen und Roh-MDI, enthaltenden Roh-MDI gegebenenfalls noch mindestens ein Antioxidans auf Phenolbasis, mindestens ein Arylphosphit oder eine Mischung dieser Stabilisatoren hinzugefügt werden. Sofern diese Stabilisatoren, die in Verbindung mit den erfindungsgemäß verwendeten Polyoxyalkylenalkohole eine zusätzliche Reduzierung der Iodfarbzahl bewirken können, Anwendung finden, werden sie zweckmäßigerweise in einer Menge von 0 bis maximal 5 Gew.-%, vorzugsweise von 0,01 bis 3 Gew.-% und insbesondere 0,1 bis 1,0 Gew.-%, bezogen auf das Gewicht des Roh-MDI, eingesetzt.

Als geeignete Antioxidantien auf Phenolbasis kommen beispielsweise in Betracht: Styrolisierte Phenole, das sind Phenole, die in 2- oder 4-Stellung oder in 2- und 4- und/oder 6-Stellung eine 1-Phenyl-ethylgruppe gebunden enthalten, Bis[2-hydroxy-5-methyl-3-tert.-butylphenyl]methan, 2,2-Bis-[4-hydroxy-phenyl]-propan, 4,4'-Dihydroxy-biphenyl, 3,3'-Dialkyl- bzw. 3,3',5,5'-Tetraalkyl-4,4'-dihydroxy-biphenyl, Bis-[4-hydroxy-2-methyl-5-tert.-butylphenyl]-sulfid, Hydrochinon, 4-Methoxy-, 4-tert.-Butoxy- oder 4-Benzyloxy-phenol, Gemische aus 4-Methoxy-2- bzw. -3-tert.-butylphenol, 2,5-Dihydroxy-1-tert.-butyl-benzol, 2,5-Dihydroxy-1,4-di-tert.-butyl-benzol, 4-Methoxy-2,6-di-tert.-butylphenol und vorzugsweise 2,6-Di-tert.-butyl-p-kresol.

Als Arylphosphite bewährt haben sich Tri-(alkylphenyl)-phosphite mit 1 bis 10 C-Atomen im Alkylrest, wie z.B. Tri-(methylphenyl)-, Tri-(ethylphenyl)-, Tri-(n-propylphenyl)-, Tri-(isopropylphenyl)-, Tri-(n-butylphenyl)-, Tri-(sek.-butylphenyl)-, Tri-(tert.butylphenyl)-, Tri-(pentylphenyl)-, Tri-(hexylphenyl)-, Tri-(2-ethyl-hexylphenyl)-, Tri-(octylphenyl)-, Tri-(2-ethyl-octylphenyl)-, Tri-(decylphenyl)-phosphit und vorzugsweise Tri-(nonylphenyl)-phosphit, und insbesondere Triphenylphosphit.

Zur Herstellung der Roh-MDI mit reduzierter Iodfarbzahl nach dem erfindungsgemäßen Verfahren werden die entsprechenden Roh-MDA zweckmäßigerweise bei einer Temperatur im Bereich von 90 bis 220°C, vorzugsweise von 120 bis 180°C, bei erhöhtem Druck, z.B. bei 1 bis 10 bar, vorzugsweise 1 bis 3 bar oder insbesondere bei Normaldruck, phosgeniert. Die bei dem erfindungsgemäßen Verfahren angewandte Temperatur liegt über der Zersetzungstemperatur der als Zwischenprodukte durch die Reaktion von Roh-MDA mit Phosgen gebildeten Carbamidsäurechloride. Einer Erhöhung des Drucks sind nur technische und gegebenenfalls sicherheitstechnische Grenzen gesetzt, wobei jedoch mit einer größeren Druckerhöhung keine Ausbeutesteigerungen mehr verbunden sind.

Nach beendeter Phosgenierung wird der Reaktionsmischung, die aus mindestens einem inerten organischen Lösungsmittel, gelöstem Roh-MDI, überschüssigem Phosgen, Chlorwasserstoff sowie Nebenprodukten der Phosgenierung besteht und die üblicherweise einen Phosgengehalt von kleiner als 20 Gew.-%, vorzugsweise von 0,01 bis 3 Gew.-% und insbesondere von 0,1 bis 2 Gew.-%, bezogen auf das Gesamtgewicht, besitzt bei einer Temperatur von 20 bis 150°C, vorzugsweise von 70 bis 120°C und insbesondere 80 bis 110°C der ein- und/oder vorzugsweise mehrwertige Polyoxyalkylenalkohol, insbesondere mit Glycerin gestartetes Polyoxypropylen-polyol einverleibt. Nach einer Verweilzeit von 0,1 bis 45 Minuten, vorzugsweise von 2 bis 25 Minuten bei einer Temperatur von 20 bis 150°C, vorzugsweise von 70 bis 120°C wird das überschüssige Phosgen bei Normaldruck und anschließend bei einer Temperatur von 30 bis 180°C, vorzugsweise von 50 bis 150°C das inerte organische Lösungsmittel oder Mischungen davon unter vermindertem Druck, z.B. bei einem Druck von 0,01 bis 100 mbar, vorzugsweise von 0,1 bis 50 mbar im wesentlichen vollständig, vorzugsweise durch Destillation, abgetrennt.

Den ein- und/oder vorzugsweise mehrwertigen Polyoxyalkylenalkoholen und/oder Reaktionsprodukten dieser Polyoxyalkylenalkohole mit Roh-MDI enthaltenden Roh-MDI's können nunmehr, sofern dies zweckdienlich erscheint, mindestens ein Antioxidans auf Phenolbasis und/oder mindestens ein Arylphosphit in einer wirksamen Menge hinzugefügt werden. Danach werden die auf diese Weise behandelten Roh-MDI zur Entchlorierung auf eine Temperatur von 100 bis 250°C, vorzugsweise von 140 bis 200°C erhitzt und bei dieser Temperatur unter einem Druck von 0,01 bis 100 mbar, vorzugsweise von 0,1 bis 20 mbar mindestens 5 Minuten und insbesondere 5 bis 45 Minuten, behandelt. Nach der Abkühlung auf 60°C wird das Roh-MDI der Zwischenlagerung zugeführt und dort weiter abkühlen gelassen.

Die nach dem erfindungsgemäßen Verfahren hergestellten Roh-MDI besitzen eine deutlich reduzierte Iodfarbzahl, üblicherweise von maximal 60 und finden Verwendung zur Herstellung von kompakten oder geschäumten Polyisocyanat-polyadditionsprodukten, vorzugsweise flexiblen, halbharten oder harten Urethan- oder Urethan- und Isocyanuratgruppen enthaltenden Schaumstoffen, die eine deutlich hellere Farbe

5

aufweisen.

Beispiele 1 bis 16

Zu einer Reaktionsmischung, die bezogen auf 100 Gew.-Teile, bestand aus
82 Gew.-Teilen Monochlorbenzol als Lösungsmittel,
8 Gew.-Teilen überschüssiges Phosgen und
10 Gew.-Teilen Roh-MDI, das seinerseits enthielt:
50 Gew.-% 4,4' -MDI,
4 Gew.-% 2,4' -MDI,
0,04 Gew.-% 2,2'-MDI und
45,96 Gew.-% Homologe mit mehr als zwei Isocyanatgruppen, bezogen auf das Roh-MDI-Gewicht, sowie nicht identifizierte Nebenkomponenten
fügte man bei einer Temperatur von 100°C bis 105°C die ein- oder mehrwertigen Polyoxyalkylenalkohole.

Die Reaktionsmischung wurde danach'in ungefähr 20 Minuten auf 140°C erwärmt und unter Normaldruck das überschüssige Phosgen innerhalb 20 Minuten mit Hilfe eines Rotationsverdampfers abdestilliert.

Die Reaktionsmischung ließ man anschließend in ungefähr 10 Minuten auf 100 bis 120°C abkühlen und destillierte in diesem Temperaturbereich unter vermindertem Druck (50 bis 10 mbar) innerhalb von ungefähr 15 Minuten das Monochlorbenzol im wesentlichen vollständig ab.

Die erhaltenen ein- und/oder mehrwertigen Polyoxyalkylenalkohole und/oder Reaktionsprodukte dieser Polyoxyalkylenalkohole mit Roh-MDI enthaltende Roh-MDI wurden nunmehr bei 180°C und 10 mbar 15 Minuten lang entchloriert.

Die eingesetzten ein- und mehrwertigen Polyoxyalkylenalkohole und ihre Mengen sowie die an den erhaltenen Roh-MDI gemessenen Iodfarbzahlen (JFZ) sind in Tabelle 1 zusammengefaßt.

Zur Bestimmung der Jodfarbzahl nach DIN 6162 wurde das Roh-MDI mit Monochlorbenzol im Volumenverhältnis 1:5 verdünnt.

In der Tabelle bedeuten:
PO :        1,2-Propylenoxideinheiten
EO :        Ethylenoxideinheiten
(Zahl):     Menge an PO- bzw. EO-Einheiten in Gew.-%, bezogen auf das Gesamtgewicht an PO- und EO-Einheiten

Tabelle 1

| Beispiel | Einsatzstoffe Zusatz ein- oder mehrwertiger Polyoxyalkylen-alkohole nach beendeter Phosgenierung | | OH-Zahl | Erhaltene Roh-MDI Eigenschaften | | |
|---|---|---|---|---|---|---|
| | Art | Menge [Gew.-% bez. auf Roh-MDI] | | JFZ (1:5) | NCO-Gehalt [Gew.-%] | Viskosität [m·Pas] |
| Roh-MDI | – | – | – | 90 | 32,1 | 50 |
| 1 | Glycerin-PO(87)-EO(13) | 5 | 35 | 50 | 30,7 | 66 |
| 2 | " | 10 | 35 | 40 | 28,5 | 88 |
| 3 | Glycerin-PO(26)-EO(74) | 4 | 42 | 50 | 30,7 | 65 |
| 4 | " | 10 | 42 | 25 | 29,0 | 108 |
| 5 | Methanol-PO | 2 | 108 | 60 | 31,2 | 53 |
| 6 | Polyoxytetramethylen-glykol | 4 | 176 | 50 | 31,1 | 47 |
| 7 | " | 10 | 176 | 20 | 28,9 | 72 |
| 8 | Glycerin-PO | 1 | 400 | 35 | 31,7 | 53 |
| 9 | " | 4 | 400 | 27 | 30,0 | 90 |
| 10 | " | 10 | 400 | 20 | 27,2 | 326 |
| 11 | " | 0,5 | 560 | 30 | 32,2 | 44 |
| 12 | " | 1 | 560 | 32 | 31,9 | 48 |
| 13 | " | 1,5 | 560 | 20 | 31,5 | 73 |
| 14 | Glycerin-EO | 1 | 749 | 28 | 31,7 | 50 |
| 15 | Glycerin-PO | 1 | 1120 | 20 | 32,0 | 48 |
| 16 | Glycerin-EO | 1 | 1240 | 23 | 31,9 | 55 |

Beispiele 17 bis 22

Man verfährt analog den Angaben der Beispiele 1 bis 16, verwendet jedoch eine Reaktionsmischung, die bezogen auf 100 Gew.-Teile, bestand aus

88,6 Gew.-Teilen Monochlorbenzol als Lösungsmittel,

0,6 Gew.-Teilen überschüssiges Phosgen und

10,8 Gew.-Teilen Roh-MDI, das seinerseits enthielt:

    50 Gew.-% 4,4'-MDI,

    4 Gew.-% 2,4'-MDI,

    0,04 Gew.-% 2,2' -MDI und

    45,96 Gew.-% Homologe mit mehr als zwei Isocyanatgruppen, bezogen auf das Roh-MDI-Gewicht, sowie nicht identifizierte Nebenkomponenten

Die verwendeten Dialkylenglykole oder Polyoxyalkylen-Triole und ihre Mengen sowie die an den erhaltenen Roh-MDI gemessenen Jodfarbzahlen sind in Tabelle 2 zusammengefaßt.

Tabelle 2

| Beispiel | Einsatzstoffe Zusatz ein- oder mehrwertiger Polyoxyalkylen-alkohole nach beendeter Phosgenierung | | | Erhaltene Roh-MDI Jodfarbzahl (1:5) |
|---|---|---|---|---|
| | Art | Menge [Gew.-% bez. auf Roh-MDI] | OH-Zahl | |
| Roh-MDI | – | – | – | 80 |
| 17 | Diethylenglykol | 0,2 | 1059 | 35 |
| 18 | Dipropylenglykol | 0,2 | 836 | 35 |
| 19 | Glycerin-PO | 0,2 | 1120 | 50 |
| 20 | Glycerin-PO | 1,0 | 1120 | 18 |
| 21 | Glycerin-EO | 1,0 | 749 | 25 |
| 22 | Glycerin-EO | 1,0 | 1240 | 19 |

**Patentansprüche**

1. Verfahren zur Herstellung von Mischungen aus Diphenylmethan-diisocyanaten und Polyphenyl-polymethylen-polyisocyanaten mit einer verminderten Iodfarbzahl durch Umsetzung der entsprechenden Mi-

schung aus
Diphenylmethan-diaminen und Polyphenyl-polymethylen-polyaminen mit Phosgen in Gegenwart mindestens eines inerten organischen Lösungsmittels bei erhöhter Temperatur,
nach beendeter Phosgenierung Abtrennung des überschüssigen Phosgens und Lösungsmittels und thermische Behandlung des Reaktionsprodukts,
dadurch gekennzeichnet, daß man der Reaktionsmischung nach beendeter Phosgenierung ein- oder mehrwertige Polyoxyalkylenalkohole oder Mischungen davon in einer wirksamen Menge einverleibt.

2.  Verfahren nach Anspruch 1, dadurch gekennzeichnet, daß die ein- oder mehrwertigen Polyoxyalkylenalkohole eine Hydroxylzahl von 20 bis 1800 besitzen.

3.  Verfahren nach Anspruch 1 oder 2, dadurch gekennzeichnet, daß man die ein- oder mehrwertigen Polyoxyalkylenalkohole oder Mischungen davon in einer Menge von 0,1 bis 10 Gew.-%, bezogen auf das Gewicht der Mischung aus Diphenylmethan-diisocyanaten und Polyphenyl-polymethylenpolyisocyanaten in der Reaktionsmischung, einverleibt.

4.  Verfahren nach einem der Ansprüche 1 bis 3, dadurch gekennzeichnet, daß die mehrwertigen Polyoxyalkylenalkohole eine Funktionalität von 2 bis 3 und eine Hydroxylzahl von 100 bis 1100 besitzen.

5.  Verfahren nach einem der Ansprüche 1 bis 4, dadurch gekennzeichnet, daß die ein- oder mehrwertigen Polyoxyalkylenalkohole aus Polyoxyethylen-, Polyoxypropylen-, Polyoxypropylen-polyoxyethylenalkoholen oder Mischungen davon bestehen.

6.  Verfahren nach einem der Ansprüche 1 bis 4, dadurch gekennzeichnet, daß man als mehrwertigen Polyoxyalkylenalkohol ein trifunktionelles Polyoxypropylen-polyol mit einer Hydroxylzahl von 380 bis 600 verwendet.

7.  Verfahren nach einem der Ansprüche 1 bis 4, dadurch gekennzeichnet, daß die mehrwertigen Polyoxyalkylenalkohole Diethylenglykol, Dipropylenglykol oder Mischungen davon sind.

8.  Verfahren nach einem der Ansprüche 1 bis 7, dadurch gekennzeichnet, daß die Reaktionsmischung einen Phosgengehalt von 0,01 bis 3 Gew.-%, bezogen auf das Gesamtgewicht, aufweist.

9.  Verfahren nach einem der Ansprüche 1 bis 8, dadurch gekennzeichnet, daß man zur Herstellung der Mischungen aus Diphenylmethan-diisocyanaten und Polyphenyl-polymethylen-polyisocyanaten als inerte organische Lösungsmittel Monochlorbenzol, Dichlorbenzole oder Mischungen davon verwendet und die eingesetzten ein- oder mehrwertigen Polyoxyalkylenalkohole in diesen Lösungsmitteln zumindest teilweise löslich sind.

10.  Verfahren nach einem der Ansprüche 1 bis 9, dadurch gekennzeichnet, daß die erhaltenen Mischungen aus Diphenylmethan-diisocyanaten und Polyphenyl-polymethylen-polyisocyanaten einen Diphenylmethandiisocyanat-Isomerengehalt von 30 bis 90 Gew.-%, einen NCO-Gehalt von 31±2 Gew.-%, jeweils bezogen auf das Gesamtgewicht, eine Viskosität von maximal 2000 m•Pa•s bei 23°C und eine Iodfarbzahl von maximal 60 besitzen.

11.  Verfahren nach einem der Ansprüche 1 bis 10, dadurch gekennzeichnet, daß man der ein- oder mehrwertige Polyoxyalkylenalkohole enthaltenden Mischung aus Diphenylmethan-diisocyanaten und Polyphenyl-polymethylenpolyisocyanaten nach der Abtrennung des überschüssigen Phosgens und des inerten organischen Lösungsmittels und vor der thermischen Behandlung des Reaktionsprodukts mindestens ein Antioxidans auf Phenolbasis in einer Menge von maximal 5 Gew.-% und/oder mindestens ein Arylphosphit in einer Menge von maximal 5 Gew.-%, jeweils bezogen auf das Gewicht der Mischung aus Diphenylmethan-diisocyanaten und Polyphenylpolymethylen-polyisocyanaten, einverleibt.

12.  Verfahren nach Anspruch 1, dadurch gekennzeichnet, daß man der Reaktionsmischung nach beendeter Phosgenierung 0,01 bis 5 Gew.-% Diethylenglykol, Dipropylenglykol oder eines trifunktionellen Polyoxypropylen-polyols, bezogen auf das Gewicht der Mischung aus Diphenylmethan-diisocyanaten und Polyphenyl-polymethylenpolyisocyanaten in der Reaktionsmischung, einverleibt, danach das über-

10

schüssige Phosgen und das inerte organische Lösungsmittel abdestilliert, der Reaktionsmischung 0 bis 5 Gew.-% Di-tert.-butyl-p-kresol und/oder Triphenylphosphit, bezogen auf das Gewicht der Mischung aus Diphenylmethan-diisocyanaten und Polyphenyl-polymethylenpolyisocyanaten, hinzufügt und danach das Reaktionsprodukt thermisch behandelt.

**Claims**

1. A process for the preparation of mixtures of diphenylmethane diisocyanates and polyphenylpolymethylene polyisocyanates of reduced iodine color number, by reacting the corresponding mixtures of

   diphenylmethanediamines and polyphenylpolymethylenepolyamines with phosgene in the presence of at least one inert organic solvent at elevated temperature,

   removing the excess phosgene and solvent when the phosgenation is complete, and

   heating the reaction product,

   which comprises incorporating monohydric or polyhydric polyoxyalkylene alcohols or mixtures thereof in an effective amount into the reaction mixture when the phosgenation is complete.

2. A process as claimed in claim 1, wherein the monohydric or polyhydric polyoxyalkylene alcohols have a hydroxyl number of from 20 to 1800.

3. A process as claimed in claim 1 or 2, wherein the monohydric or polyhydric polyoxyalkylene alcohols or mixtures thereof are incorporated into the reaction mixture in an amount of from 0.1 to 10% by weight, based on the weight of the mixture of diphenylmethane diisocyanates and polyphenylpolymethylene polyisocyanates.

4. A process as claimed in claim 1 or 2 or 3, wherein the polyhydric polyoxyalkylene alcohols have a functionality of from 2 to 3 and a hydroxyl number of from 100 to 1100.

5. A process as claimed in claim 1 or 2 or 3 or 4, wherein the monohydric or polyhydric polyoxyalkylene alcohols comprise polyoxyethylene alcohol, polyoxypropylene alcohol or polyoxypropylenepolyoxyethylene alcohol, or mixtures thereof.

6. A process as claimed in claim 1 or 2 or 3 or 4, wherein the polyhydric polyoxyalkylene alcohol used is a trifunctional polyoxypropylene-polyol having a hydroxyl number of from 380 to 600.

7. A process as claimed in claim 1 or 2 or 3 or 4, wherein the polyhydric polyoxyalkylene alcohols are diethylene glycol, dipropylene glycol or mixtures thereof.

8. A process as claimed in claim 1 or 2 or 3 or 4 or 5 or 6 or 7, wherein the reaction mixture contains from 0.01 to 3% by weight of phosgene, based on the total weight.

9. A process as claimed in claim 1 or 2 or 3 or 4 or 5 or 6 or 7 or 8, wherein the inert organic solvent used for the preparation of the mixtures of diphenylmethane diisocyanates and polyphenylpolymethylene polyisocyanates is monochlorobenzene or a dichlorobenzene, or a mixture thereof, and the monohydric or polyhydric polyoxyalkylene alcohols employed are at least partially soluble in this solvent.

10. A process as claimed in claim 1 or 2 or 3 or 4 or 5 or 6 or 7 or 8 or 9, wherein the resulting mixtures of diphenylmethane diisocyanates and polyphenylpolymethylene polyisocyanates contain from 30 to 90% by weight of diphenylmethane diisocyanate isomers, and 31±2% by weight of NCO, in each case based on the total weight, and have a maximum viscosity of 2000 mPa•s at 23°C and a maximum iodine color number of 60.

11. A process as claimed in claim 1 or 2 or 3 or 4 or 5 or 6 or 7 or 8 or 9 or 10, wherein at least one phenol-based antioxidant in a maximum amount of 5% by weight and/or at least one aryl phosphite in a maximum amount of 5% by weight, in each case based on the weight of the mixture of diphenylmethane diisocyanates and polyphenylpolymethylene polyisocyanates, are incorporated into the monohydric or polyhydric polyoxyalkylene alcohol-containing mixture of diphenylmethane diisocyanates and polyphenylpolymethylene polyisocyanates after the excess phosgene and the inert organic solvent have been removed and before the reaction product has been heated.

12. A process as claimed in claim 1, wherein from 0.01 to 5% by weight of diethylene glycol, dipropylene glycol or a trifunctional polyoxypropylene-polyol, based on the weight of the mixture of diphenylmethane diisocyanates and polyphenylpolymethylene polyisocyanates in the reaction mixture, is incorporated into the reaction mixture when the phosgenation is complete, the excess phosgene and the inert organic solvent are then removed by distillation, from 0 to 5% by weight of di-tert-butyl-p-cresol and/or triphenyl phosphite, based on the weight of the mixture of diphenylmethane diisocyanates and polyhenylpolymethylene polyisocyanates, is added to the reaction mixture, and the reaction product is then heated.

**Revendications**

1. Procédé de préparation de mélanges de diisocyanates de diphénylméthane et de polyphénylpolyméthylènepolyisocyanates à indice de coloration d'iode réduit, par la réaction des mélanges correspondants constitués
   de diphénylméthanediamines et de polyphénylpolyméthylènepolyamines avec le phosgène, en présence d'au moins un solvant organique, inerte, à température élevée,
   une fois le phosgénation achevée, l'élimination du solvant et du phosgène excédentaire et traitement thermique du produit de réaction,
   caractérisé en ce qu'une fois la phosgénation achevée, on incorpore au mélange réactionnel des polyoxyalkylènealcools monohydroxylés ou polyhydroxylés, ou des mélanges de ceux-ci, en une proportion efficace.

2. Procédé suivant la revendication 1, caractérisé en ce que les polyoxyalkylènealcools monohydroxylés ou polyhydroxylés possèdent un indice d'hydroxyle de 20 à 1800.

3. Procédé suivant la revendication 1 ou 2, caractérisé en ce que les polyoxyalkylènealcools monohydroxylés ou polyhydroxylés, ou des mélanges de ces derniers, sont incorporés au mélange réactionnel en une proportion de 0,1 à 10% en poids, par rapport au poids du mélange de diisocyanates de diphénylméthane et de polyphénylpolyméthylènepolyisocyanates.

4. Procédé suivant l'une quelconque des revendications 1 à 3, caractérisé en ce que les polyoxyalkylènealcools polyhydroxylés possèdent une fonctionnalité de 2 à 3 et un indice d'hydroxyle de 100 à 1100.

5. Procédé suivant l'une quelconque des revendications 1 à 4, caractérisé en ce que les polyoxyalkylènealcools monohydroxylés ou polyhydroxylés se composent de polyoxyéthylène-, polyoxypropylène-, polyoxypropylènepolyoxyéthylènealcools, ou des mélanges de ceux-ci.

6. Procédé suivant l'une quelconque des revendications 1 à 4, caractérisé en ce que l'on utilise, à titre de polyoxyalkylènealcool polyhydroxylé, un polyoxypropylènepolyol trifonctionnel d'un indice d'hydroxyle de 380 à 600.

7. Procédé suivant l'une quelconque des revendications 1 à 4, caractérisé en ce que les polyoxyélkylène alcools polyhydroxylés sont le diéthylèneglycol, le dipropylèneglycol, ou des mélanges de ceux-ci.

8. Procédé suivant l'une quelconque des revendications 1 à 7, caractérisé en ce que le mélange réactionnel présente une teneur en phosgène de 0,01 à 3% en poids, par rapport au poids total.

9. Procédé suivant l'une quelconque des revendications 1 à 8, caractérisé en ce qu'en vue de la préparation des mélanges de diisocyanates de diphénylméthane et de polyphénylpolyméthylènepolyisocyanates, on utilise, à titre de solvants organiques et inertes, le monochlorobenzène, des dichlorobenzènes, ou des mélanges de ceux-ci et les polyoxyalkylènealcools monohydroxylés ou polyhydroxylés mis en oeuvre sont au moins partiellement solubles dans ces solvants.

**10.** Procédé suivant l'une quelconque des revendications 1 à 9, caractérisé en ce que les mélanges obtenus de diisocyanates de diphénylméthane et de polyphénylpolyméthylènepolyisocyanatespossèdent une teneur en isomères de diisocyanate de diphénylméthane de 30 à 90% en poids, une teneur en NCO de 31±2% en poids, à chaque fois rapportés au poids total, une viscosité d'au maximum 2000 mPa.s à 23°C et un indice de coloration d'iode de 60 au maximum.

**11.** Procédé suivant l'une quelconque des revendications 1 à 10, caractérisé en ce que l'on incorpore au mélange de diisocyanates de diphénylméthane et de polyphénylpolyméthylènepolyisocyanates contenant les polyoxyalkylènealcools monohydroxylés ou polyhydroxylés, après la séparation du solvant organique et inerte et du phosgène excédentaire et avant le traitement thermique du produit réactionnel, au moins un antioxydant à base de phénol en une proportion d'au moins 5% en poids et au moins un phosphite d'aryle en une proportion d'au maximum 5% en poids, rapportés à chaque fois au poids du mélange de diisocyanates de diphénylméthane et de polyphénylpolyméthylènepolyisocyanates.

**12.** Procédé suivant la revendication 1, caractérisé en ce qu'une fois la phosgénation achevée, on incorpore de 0,01 à 5% en poids de diéthylèneglycol, de dipropylèneglycol, ou d'un polyoxypropylènepolyol trifonctionnel, par rapport au poids du mélange des diisocyanates de diphénylméthane et des polyphénylpolyméthylènepolyisocyanates, au mélange réactionnel, puis on élimine par distillation le solvant organique et inerte et le phosgène excédentaire par distillation, on ajoute au mélange réactionnel de 0 à 5% en poids de di-tert-butyl-p-crésol et/ou de phosphite de triphényle, par rapport au poids du mélange des diisocyanates de diphénylméthane et des polyphénylpolyméthylènepolyisocyanates, puis on soumet le produit réactionnel à un traitement thermique.